# EUROPÄISCHE PATENTANMELDUNG

(11) **EP 0 569 682 A1**
(43) Veröffentlichungstag der Anmeldung: **18.11.1993**
(21) Anmeldenummer: 93103986.1
(22) Anmeldetag: 11.03.1993
(51) Int. Cl.: C07H 15/04

(54) **Verfahren zur Herstellung von Alkylpolyglycosiden**

(30) Priorität: 11.05.1992 DE 4215431
(71) Anmelder: HÜLS AKTIENGESELLSCHAFT, D-45764 Marl (DE)
(72) Erfinder: Balzer, Dieter, Dr., W-4358 Haltern (DE); Ripke, Norbert, Dr., W-4358 Haltern (DE)

(57) **Zusammenfassung**

Die Erfindung betrifft die Herstellung von grenzflächenaktiven Alkylpolyglycosiden durch Glycosidierung und Umglycosidierung. Dadurch, daß man die Umglycosidierung in Gegenwart Phosphor enthaltender Mineralsäuren, ihrer Alkali- oder Erdalkalisalze durchführt, werden farblich ansprechende Produkte mit verbesserter Wassermischbarkeit erhalten.

## Beschreibung

Die Erfindung betrifft ein Verfahren zur Herstellung von farblich ansprechenden Alkylpolyglycosiden mit einer Alkylgruppe mit 8 bis 24 C-Atomen, bei dem Saccharide glycosidiert und die erhaltenen Produkte umglycosidiert werden.

Alkylpolyglycoside sind ungiftige und leichte abbaubare oberflächenaktive Stoffe. Sie werden deshalb als Tenside in Wasch- und Reinigungsmitteln und in Shampoos als Emulgatoren und Dispergatoren verwendet. Sie haben aber nur dann die gewünschten Grenzflächeneigenschaften, wenn die Alkylgruppen mindestens 8 C-Atome aufweisen.

Alkylpolyglycoside mit langkettigen Alkylresten können ganz oder teilweise aus nachwachsenden Rohstoffen hergestellt werden. Für Anwendungen im Haushalt und im Kosmetikbereich müssen diese Produkte hohen ästhetischen Ansprüchen genügen. Daher sind Verfahren interessant, nach denen Alkylpolyglycoside in transparenten, möglichst ungefärbten wäßrigen Lösungen hergestellt werden können.

Die Alkylpolyglycoside werden im allgemeinen aus Sacchariden und Alkoholen durch Glycosidierung und Umglycosidierung hergestellt. Bei einem zweistufigen Verfahren wird beispielsweise zunächst durch Glycosidierung mit n-Butanol ein n-Butylglycosid und daraus durch Umglycosidierung mit einem langkettigen Alkohol das gewünschte langkettigen Alkylpolyglycosid hergestellt. Die Produkte, die man dabei erhält, sind jedoch, wenn keine zusätzlichen Maßnahmen ergriffen werden, dunkel gefärbt und nicht in jedem Verhältnis mit Wasser mischbar.

Die Farbe kann nach US 4 742 918 durch katalytische Hydrierung verbessert werden.

Bei der Herstellung von langkettigen Alkylsacchariden können nach US 4 465 828 auch die Hydroxylpolycarbonsäuren Citronensäure, Weinsäure und Äpfelsäure zur Farbverbesserung beitragen.

Nach EP 0 077 167 können bei der direkten Umsetzung von Glucose mit langkettigen Alkoholen Reduktionsmittel, wie hypophosphorige Säure oder schwefelige Säure, zugesetzt werden, wodurch Farbverbesserungen erzielt werden.

Es sind auch vorbeugende Maßnahmen bekannt. So erhält man nach EP 0 102 558 farblich verbesserte C₃- bis C₅-Alkylglucoside, wenn man die Glucosidierung in Gegenwart eines Alkalisalzes einer Borsäure durchführt.

Nach EP 0 165 721 kann die Farbe der Produkte durch mehrstufige Bleichung mit Wasserstoffperoxid verbessert und durch Zusatz von Schwefeldioxid freisetzenden Verbindungen stabilisiert werden.

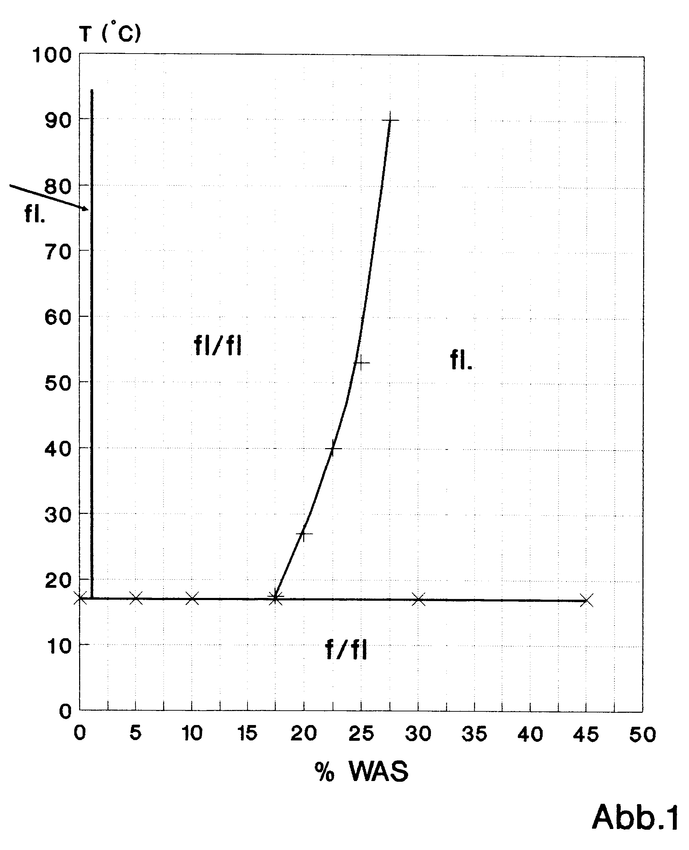

Nach allen beschriebenen Verfahren erhält man Alkylpolyglucoside, die besonders in den Fällen der anwendungstechnisch sehr interessanten Alkylketten mit 12 bis 18 C-Atomen nur begrenzt mit Wasser mischbar sind. So zeigt das Phasendiagramm eines C₁₂/C₁₄-Alkylpolyglycosides eine breite Mischungslücke im Konzentrationsbereich von etwa 1 bis 17 % bei 18 °C und von etwa 1 bis 30 % bei 90 °C (vgl. Abbildung 1). Beim Verdünnen einer im Handel üblichen etwa 50%igen wäßrigen Lösung von waschaktiver Substanz (Alkylpolyglycosid) mit Wasser tritt also im oben angegebenen Konzentrationsbereich eine trübe Lösung auf. Es entstehen zwei Phasen, die zusätzlich auch noch sehr schnell separieren. Für den Anwender hat diese schlechte Wassermischbarkeit den Nachteil, daß die Produkte nur sehr schwer genau dosiert werden können.

Eine verbesserte Dosierung von mit Wasser nicht mischbaren Produkten kann allgemein durch Zusatz von Emulgatoren oder Lösungsvermittler erfolgen. Derartige Verbindungen sind beispielsweise Tenside, wie Alkylbenzolsulfonate, Alkansulfonate, Alkylphenoloxethylate, Fettalkoholoxethylate, Alkohole oder Glykole. Diese Substanzen sind zum Teil für kosmetische Anwendungen nicht zugelassen. Außerdem entsprechen sie teilweise toxikologisch oder ökologisch nicht den Erfordernissen eines modernen Produkts.

Aufgabe der vorliegenden Erfindung ist es daher, die Farbe und gleichzeitig die Wassermischbarkeit der Alkylpolyglycoside mit langkettigen Alkylresten zu verbessern, ohne dabei auf den Zusatz von anderen Tensiden oder Lösungsvermittlern zurückgreifen zu müssen.

Die Aufgabe wird erfindungsgemäß dadurch gelöst, daß man die Umglycosidierung in Gegenwart Phosphor enthaltender Mineralsäuren, ihrer Alkalioder ihrer Erdalkalisalze durchführt.

Es überrascht, daß die Mischbarkeit der Alkylpolyglycoside mit Wasser durch die erfindungsgemäßen Maßnahmen beträchtlich verbessert wird. Die Mischungslücke wird verkleinert, zu höheren Temperaturen verschoben oder sogar ganz vermieden.

Geeignete Mineralsäuren sind vor allem Phosphorsäure, phosphorige Säure und hypophosphorige Säure. Als Alkalisalze kommen zum Beispiel die Lithium-, Natrium- und Kaliumsalze in Betracht. Beispiele für Erdalkalisalze sind Magnesium-, Calcium- und Bariumsalze. Vorzugsweise verwendet man die hypophosphorige Säure, ihre Alkali- und Erdalkalisalze. Dabei werden Alkalihypophosphite ganz besonders bevorzugt.

Die genannten Phosphor enthaltenden Verbindungen werden vorzugsweise in Mengen von 0,1 bis 1 Gewichtsprozent, bezogen auf die zur Glycosidierung eingesetzte Reaktionsmischung, zugemischt. Gehalte von 0,15 bis 0,5 Gewichtsprozent werden dabei ganz besonders bevorzugt. Dabei können diese Phosphor enthaltenden Verbindungen zur Glycosidierung oder nach der Glycosidierung zur Umglycosidierung zugesetzt werden.

Für die Glycosidierung werden im allgemeinen Saccharid, kurzkettiger Alkohol mit 1 bis 6 C-Atomen und saurer Katalysator gemischt und dann erwärmt, wobei die Reaktion unter Wasserbildung beginnt.

Als Saccharide können Monosaccharide, wie Glucose, Mannose, Gulose, Galaktose oder Talose, aber auch Di- und Oligosaccharide mit bis zu 10 Saccharideinheiten verwendet werden. Die Einheiten können 1,2-, 1,3-, 1,4-oder 1,6-verknüpft sein. Es können α- oder β-Verknüpfungen vorliegen. Die Ketten können linear oder verzweigt sein. Vorzugsweise wird Glucose eingesetzt. Man kann auch wasserhaltige Produkte in die Reaktion einführen.

Als kurzkettige Alkohole können beispielsweise Ethanol, Propanol, Butanol, Pentanol, aber auch Glykole, wie Ethylenglykol und Propylenglykol verwendet werden.

Als saure Katalysatoren kann man Mineralsäuren, wie Schwefel- oder Salzsäure, einsetzen. Gut geeignet sind auch organische Säuren, wie beispielsweise Aryl-, Alkyl- oder Aralkylsulfonsäuren.

Die Reaktion wird vorzugsweise bei einer Produkttemperatur von 60 bis 160 °C durchgeführt. Dabei wird ein Temperaturbereich von 80 bis 120 °C besonders bevorzugt. Die Reaktion kann bei Normaldruck, bei leichtem Unterdruck und auch bei Überdruck ausgeführt werden.

Die bei der Glycosidierung gewonnenen Alkylglycoside mit kurzkettigen Alkylgruppen werden anschließend durch Umglycosidierung mit langkettigen Alkoholen mit 8 bis 24 C-Atomen zu Alkylpolyglycosiden umgesetzt. Die Umglycosidierung kann nach bekannten Methoden erfolgen.

Die bei der Umglycosidierung eingesetzten Alkohole können linear sein. Sie können aber auch Verzweigungen enthalten. Sie können gesättigt sein oder auch olefinische Doppelbindungen enthalten. Man kann natürliche oder synthetische Fettalkohole oder Fettalkoholgemische einsetzen. Als Beispiele seien Decanol, 10-Undecen-1-ol, Dodecanol, Myristylalkohol und Stearylalkohol genannt. Vorzugsweise enthalten die Alkohole 10 bis 18 C-Atome.

Die hergestellten Alkylpolyglycoside weisen einen mittleren Polymerisationsgrad von 1 bis 10 auf. Dabei werden niedrige mittlere Polymerisationsgrade von 1,3 bis 5 bevorzugt.

Durch das erfindungsgemäße Verfahren erhält man toxikologisch und ökologisch unbedenkliche, farblich ansprechende - 50%ige Lösungen in Wasser weisen eine Iodfarbzahl (IFZ) von unter 20 auf - und außerdem mit Wasser verbessert mischbare Alkylpolyglycoside mit Tensideigenschaften. Teilweise werden Produkte erhalten, die mit Wasser in beliebigen Mengenverhältnissen mischbar sind. Die Produkte sind deshalb für den Einsatz in Wasch- und Reinigungsmitteln, in Shampoos oder Duschgelen besonders gut geeignet.

Die folgenden Beispiele sollen die Erfindung verdeutlichen.

### Vergleichsbeispiel A

In einem 4-1-Rührkessel mit aufgesetzter Kolonne und Wasserabscheider werden 750 g Butylglucosid in 3 l Butanol bei 118 °C vorgelegt. Das Gemisch wird gerührt und gleichzeitig mit einem Volumenstrom von 18 l/h durch einen Fallfilmverdampfer (0,2 m² Fläche, 160 °C Öltemperatur) gepumpt und in den Rührkessel zurückgeleitet. Zu dem umgewälzten Produkt werden vor dem Eintritt in den Verdampfer pro Stunde 0,75 l Glucosesirup (60%ig in Wasser), 9 g H₂SO₄ und 2,25 l Butanol zugegeben.

Produkttemperatur am Ausgang des Verdampfers: 116 °C
SZ im Rührkessel: 2 mg KOH/g

Butanol und Wasser werden abdestilliert und am Wasserabscheider getrennt. Butanol wird zurückgeführt.

Nach einer Reaktionszeit von 1,25 Stunden bei 118 °C (Produkttemperatur im Kessel) und Normaldruck ist die Reaktion beendet.

IFZ von Butylglucosid: 60 bis 80
(aus einer 35%igen Lösung in Butanol)

Zur anschließenden Umglycosidierung werden 2 kg Butylglucosid (IFZ: 60 bis 80) und weitere 12,5 g H₂SO₄ bei 116 °C und 400 hPa mit 37 l einer Mischung aus 70 % Dodecanol und 30 % Tetradecanol umgesetzt. Butanol wird destillativ entfernt. Nach ca. 2 Stunden ist die Reaktion beendet. Danach wird neutralisiert, worauf überschüssiger Alkohol destillativ entfernt wird. Der erhaltene Feststoff wird in Wasser gelöst und mit Ozon gebleicht. Man erhält ein fellfarbenes Alkylpolyglucosid.

Mittlerer Polymerisationsgrad: 1,65
IFZ: 3 bis 5 (aus einer 50%igen wäßrigen Lösung)

Das Produkt ist bei 20 °C mit Wasser nicht voll mischbar. Im Bereich einer 1,5 bis 18%igen wäßrigen Mischung tritt eine Mischungslücke auf (vgl. Abbildung 1).

### Beispiel 1

Es wird verfahren wie im Vergleichsbeispiel A, wobei dem Reaktionsgemisch der Glycosidierungsreaktion, bezogen auf den Reaktionsansatz, 0,15 Gewichtsprozent Natriumhypophosphit zusammen mit dem Katalysator zugegeben werden.

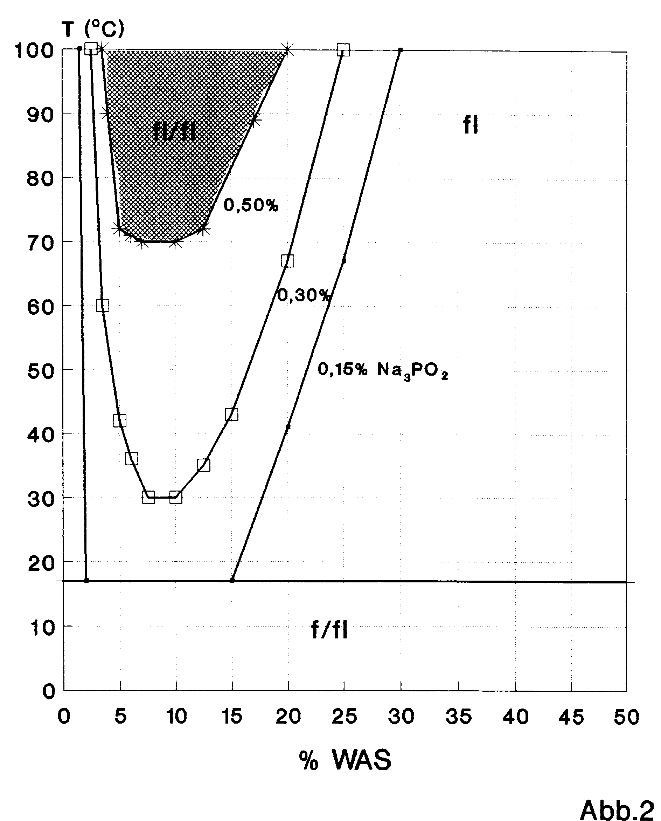

Das so hergestellte Alkylpolyglucosid weist mit Wasser bei 20 °C eine Mischungslücke zwischen etwa 2 und 16 % auf (vgl. Abbildung 2).

### Beispiel 2

Es wird verfahren wie im Vergleichsbeispiel A, wobei dem Reaktionsgemisch der Glycosidierungsreaktion, bezogen auf den Reaktionsansatz, 0,3 Gewichtsprozent Natriumhypophosphit zusammen mit dem Katalysator zugegeben werden.

Das so hergestellte Alkylpolyglucosid ist bei 20 °C mit Wasser unbegrenzt mischbar. Ein unterer kritischer Entmischungspunkt liegt bei 30 °C (vgl.

Abbildung 2).

### Beispiel 3

Es wird verfahren wie im Vergleichsbeispiel A, wobei dem Reaktionsgemisch der Glycosidierungsreaktion, bezogen auf den Reaktionsansatz, 0,5 Gewichtsprozent Natriumhypophosphit zusammen mit dem Katalysator zugegeben werden.

Das so hergestellte Alkylpolyglucosid hat in Gegenwart von Wasser einen unteren kritischen Entmischungspunkt von 69 °C (vgl. Abbildung 2).

### Beispiel 4

Es wird verfahren wie im Vergleichsbeispiel A, wobei dem Reaktionsgemisch der Glycosidierungsreaktion, bezogen auf den Reaktionsansatz, 0,8 Gewichtsprozent Natriumhypophosphit zusammen mit dem Katalysator zugegeben werden.

Das so hergestellte Alkylpolyglucosid hat in Gegenwart von Wasser einen unteren kritischen Entmischungspunkt von 90 °C.

In Abbildung 1 ist das Phasendiagramm von C₁₂/C₁₄-Alkylpolyglucosid (Vergleichsbeispiel A) mit Wasser dargestellt. Dabei bedeuten WAS waschaktive Substanz (C₁₂/C₁₄-Alkylpolyglucosid), fl einphasig flüssig, fl/fl zweiphasig flüssig-flüssig und f/fl zweiphasig fest-flüssig.

Abbildung 2 enthält die entsprechenden Phasendiagramme von C₁₂/C₁₄-Alkylpolyglucosiden (Beispiele 1 bis 3), die in Gegenwart der angegebenen Mengen an Natriumhypophosphit hergestellt wurden, mit Wasser.

## Patentansprüche

1. Verfahren zur Herstellung von Alkylpolyglycosiden, deren Alkylgruppen 8 bis 24 C-Atome aufweisen, aus Sacchariden und Alkoholen durch Glycosidierung und Umglycosidierung,
dadurch gekennzeichnet,
daß man die Umglycosidierung in Gegenwart Phosphor enthaltender Mineralsäuren, ihrer Alkali- oder Erdalkalisalze durchführt.

2. Verfahren nach Anspruch 1,
dadurch gekennzeichnet,
daß der Gehalt an Phosphor enthaltenden Mineralsäuren, ihrer Alkali- oder Erdalkalisalze 0,1 bis 1 Gewichtsprozent, bezogen auf die zur Glycosidierung eingesetzte Reaktionsmischung, beträgt.

3. Verfahren nach Anspruch 2,
dadurch gekennzeichnet,
daß der Gehalt 0,15 bis 0,5 Gewichtsprozent beträgt.

4. Verfahren nach Anspruch 1,
dadurch gekennzeichnet,
daß in Gegenwart von hypophosphoriger Säure, ihrer Alkali- oder Erdalkalisalze umglycosidiert wird.

5. Verfahren nach Anspruch 4,
dadurch gekennzeichnet,
daß in Gegenwart von Alkalihypophosphiten umglycosidiert wird.

6. Verfahren nach Anspruch 1,
dadurch gekennzeichnet,
daß man die Phosphor enthaltenden Mineralsäuren, ihre Alkali- oder Erdalkalisalze zur Umglycosidierung zusetzt.
